# EUROPEAN PATENT SPECIFICATION

(11) **EP 4 273 133 B1**
(45) Date of publication and mention of the grant of the patent: **18.12.2024**
(21) Application number: 21921796.5
(22) Date of filing: 28.01.2021
(51) Int. Cl.: C07D 309/32

(54) **METHOD FOR PREPARING 6-HYDROXY-6 (HYDROXYMETHYL)-2H-PYRAN-3(6H)-ONE BY MEANS OF CATALYTIC OXIDATION**
VERFAHREN ZUR HERSTELLUNG VON 6-HYDROXY-6 (HYDROXYMETHYL)-2H-PYRAN-3(6H)-ON DURCH KATALYTISCHE OXIDATION
PROCÉDÉ DE PRÉPARATION DE 6-HYDROXY-6 (HYDROXYMÉTHYL)-2H-PYRAN-3(6H)-ONE PAR OXYDATION CATALYTIQUE

(43) Date of publication of application: 08.11.2023
(73) Proprietor: Ningbo Institute of Materials Technology & Engineering, Chinese Academy of Sciences, Ningbo, Zhejiang 315201 (CN)
(72) Inventor: ZHANG, Jian, Ningbo, Zhejiang 315201 (CN); HAO, Panpan, Ningbo, Zhejiang 315201 (CN); LI, Zhenyu, Ningbo, Zhejiang 315201 (CN); HAN, Yingying, Ningbo, Zhejiang 315201 (CN)
(74) Representative: Bayramoglu et al.
(86) International application number: PCT/CN2021/074150
(87) International publication number: WO 2022/160177

(56) References cited:
- CN-A- 109 180 624
- CN-A- 111 548 334
- ADGER BRIAN M. ET AL: "Oxidation of Furans with Dimethyldioxirane", J. CHEM. SOC., CHEM. COMMUN.,, 1 January 1991 (1991-01-01), XP093124005, Retrieved from the Internet <URL:https://doi.org/10.1039/C39930001220> [retrieved on 20240125]
- KUO YUEH-HSIUNG ET AL: "Preparation of 6-Hydroxy-2H-pyran-3(6H)-one from 2-Furylcarbinol by Photooxidation. Synthesis of a Pheromone of Vespa orientalis", HETEROCYCLES, vol. 31, no. 11, 1 January 1990 (1990-01-01), JP, pages 1941 - 1949, XP093124008, ISSN: 0385-5414, Retrieved from the Internet <URL:https://dx.doi.org/10.3987/COM-90-5433> DOI: 10.3987/COM-90-5433
- GELMINI ANDREA, ALBONETTI STEFANIA, CAVANI FABRIZIO, CESARI CRISTIANA, LOLLI ALICE, ZANOTTI VALERIO, MAZZONI RITA: "Oxidant free one-pot transformation of bio-based 2,5-bis-hydroxymethylfuran into α-6-hydroxy-6-methyl-4-enyl-2H-pyran-3-one in water", APPLIED CATALYSIS B. ENVIRONMENTAL, ELSEVIER, AMSTERDAM, NL, vol. 180, 1 January 2016 (2016-01-01), AMSTERDAM, NL , pages 38 - 43, XP055954410, ISSN: 0926-3373, DOI: 10.1016/j.apcatb.2015.06.003
- WAHLEN JOOS, BART MOENS, DIRK E. DE VOS, PAUL L. ALSTERS, PIERRE A. JACOBS: "Titanium Silicalite 1 (TS-1) Catalyzed Oxidative Transformations of Furan Derivatives with Hydrogen Peroxide", ADVANCED SYNTHESIS AND CATALYSIS, vol. 346, no. 2-3, 29 March 2004 (2004-03-29), pages 333 - 338, XP055954408, ISSN: 1615-4150, DOI: 10.1002/adsc.200303185
- JIANG YONGQIANG, SUN JIANJUN, DU DONGMEI, ZHOU FUXIANG: "Synthesis of Ethylmaltol Utilizing TS-1", JOURNAL OF BEIJING UNIVERSITY OF CHEMICAL TECHNOLOGY (NATURAL SCIENCE EDITION), vol. 37, no. 4, 31 December 2010 (2010-12-31), pages 6 - 11, XP055954404, ISSN: 1671-6639, DOI: 10.13543/j.cnki.bhxbzr.2010.04.007
- FALENCZYK CAROLIN, PÖLLOTH BENJAMIN, HILGERS PETRA, KÖNIG BURKHARD: "Mechanochemically Initiated Achmatowicz Rearrangement", SYNTHETIC COMMUNICATIONS, TAYLOR & FRANCIS INC., US, vol. 45, no. 3, 1 February 2015 (2015-02-01), US , pages 348 - 354, XP055954405, ISSN: 0039-7911, DOI: 10.1080/00397911.2014.963624

## Description

### TECHNICAL FIELD

The present application relates to a method for preparing a biological active molecule 6-hydroxy-6(hydroxymethyl)-2*H*-pyran-3(6*H*)-one by catalytically oxidizing 2,5-furan dimethanol using titanium silicalite, belonging to the technical field of chemical synthesis.

### BACKGROUND

With the continuous consumption of fossil resources and the increasingly serious environmental pollution, biomass and its derivatives are functionalized, gradually applied to modern organic synthesis and industrial production of bulk and fine chemical through a variety of reaction ways (such as oxidation, reduction and amination), and are expected to become a sustainable substitute for petroleum based chemicals. Where, Achmatowicz rearrangement reaction is an epoxidation reaction which can oxidize furan derivatives (alkyl substituted furans, furfuryl alcohols, alkyl substituted furfuryl alcohols, etc.) to more complex and functional 6-hydroxy-2*H*-pyran-3(6*H*)-one that is more complex in structure and has more functionalization, including 6-hydroxy-6(hydroxymethyl)-2*H*-pyran-3(6*H*)-one. These pyrones contain a variety of functional groups and can be further converted, are extremely valuable synthesis intermediates, play a crucial role in synthesis of antibacterial agents, monosaccharides and their analogues, natural products and drugs (RSC Adv., 2016, 6, 111564-111598).

Pyrone is prepared by Achmatowicz rearrangement reaction. The most commonly used oxidants are N-bromosuccinimide (NBS) and me-chloroperoxybenzoic acid (m-CPBA). However, the use of these oxidants will produce quantitative organic byproducts of m-chlorobenzoic acid or succinimide, leading to a reduction in product purity, and therefore it is required to timely column chromatographic separation (Green Chem., 2019, 21, 64-68). Other reported catalytic systems include the combination of acetylacetone vanadium oxide (VO(acac)₂) or tetra(isopropoxy) titanium (Ti(Oi-Pr)₄) and tertbutyl hydroperoxide (TBHP) (Org. Lett., 2013, 15, 5088-5091), potassium bromide (KBr)/potassium peroxymonosulfonate (Oxone) (J. Org. Chem., 2016, 81, 4847-4855), bromine (Br₂)/methanol (MeOH) (J. Org. Chem., 1988, 53, 1607-1611), metal complex ([In(COD)Cl]₂)/benzyl alcohol (Adv. Synth. Catalog. 2018, 360, 595-599), etc., almost all the synthesis processes involved are homogeneous reactions, and homogeneous catalysts are difficult to separate from liquid reaction products, more attentions are paid to separation problems especially when noble metal complexes are used as catalysts, otherwise it is not economical and pollutes the product, thereby affecting the next step of reaction. In addition, the reaction system usually requires organic solvents, which reduces the "green index" of the reaction. However, the yield of 6-hydroxy-6(hydroxymethyl)-2*H*-pyran-3(6*H*)-one obtained by ball milling under solvent-free conditions is not ideal, only 45%-63% (Synth. Commun., 2015, 45, 348-354). With the increasing concern to environmental and energy issues in the field of green and sustainable technology, the development of an environmental-friendly, efficient and high-atom-economy synthesis method has become an urgent demand for the preparation of 6-hydroxy-6(hydroxymethyl)-2*H-*pyran-3(6*H*)-one.

Falenczyk Carolin, Polloth Benjamin, Hilgers Petra, Konig Burkhard: "Mechanochemically Initiated Achmatowicz Rearrangement" The Achmatowicz rearrangement converts furfuryl alcohols, obtainable from renewable carbohydrates, into 6-hydroxy-2H-pyrane-3(6H)-ones, which are versatile intermediates for organic synthesis. The first examples of a solvent-free mechanochemical Achmatowicz rearrangement are described here. Furfuryl alcohols were prepared from furfurals using mechamnochemically initiated reductions and Reformatsky reactions. Mechamochemical reaction conditions for the Achmatowicz rearramgement of the obtained furfuryl alcohols were optimized and applied to a series of derivatives, yielding the corresponding rearrangement products in yields of 39 to 95%.

Adger Brian M.ET AL: "Oxidation of Furans with Dimethyldioxirane" Dimethyldioxirane reacts rapidly at room temperature in acetone with a variety of furans, furnishing in high yield products of oxidative ring opening and, with 2-furanmethanol, 2*H*-pyran-3(6*H*)-one via subsequent ring closure.

Kuo Yueh-Hsiung ET AL: "Preparation of 6-Hydroxy-2H-pyran-3(6H)-one from 2-Furylcarbinol by Photooxidation. Synthesis of a Pheromone of Vespa orientalis" Photoxidation of 2-furylcarbinols followed by reduction with triphenyl-phosphine afforded 6-hydroxy-2H-pyran-3(6H)-one in excellent yield. The method was applied to synthesis of 6-undecyltetrahydro-2-pyrone, a pheromone of Vespa orientalis.

### SUMMARY

The main objective of the present application is to provide a method for preparing a biological active molecule 6-hydroxy-6(hydroxymethyl)-2*H*-pyran-3(6*H*)-one by catalytically oxidizing 2,5-furan dimethanol using titanium silicalite, which is mild in reaction condition, green and economic and efficient, thereby overcoming the defects in the prior art.

In order to realize the objective of the foregoing invention, the technical solution adopted by the present application includes:

The embodiment of the present application provides a method for preparing 6-hydroxy-6(hydroxymethyl)-2*H*-pyran-3(6*H*)-one through catalytic oxidization, comprising:

catalytically oxidizing 2,5-furan dimethanol for 20 min-120 min at 15°C-100°C by using titanium silicalite as a catalyst, hydrogen peroxide as an oxidant and water as a reaction medium so as to obtain 6-hydroxy-6(hydroxymethyl)-2*H*-pyran-3(6*H*)-one.

In some embodiments, the method for preparing 6-hydroxy-6(hydroxymethyl)-2*H*-pyran-3(6*H*)-one through catalytic oxidization comprises: evenly mixing 2,5-furan dimethanol, titanium silicalite, hydrogen peroxide and water, and stirring the obtained reaction system to react for 20 min-60 min at 15°C-70°C, so as to obtain 6-hydroxy-6(hydroxymethyl)-2*H*-pyran-3(6*H*)-one through one-step catalytic oxidization.

In some embodiments, a silica-to-titanium ratio in the titanium silicalite is (15-80): 1.

Compared with the prior art, the present application at least has the beneficial effects:
1) The present application provides a method for synthesizing 6-hydroxyl-6(hydroxymethyl)-2*H*-pyran-3(6*H*)-one by one-step efficient catalytic oxidization of 2,5-furan dimethanol, which uses a multiphase catalyst titanium silicalite and a cheap and environmental-friendly oxidant (hydrogen peroxide) and directly uses water as a reaction medium, thereby realizing a cleaner and more economical catalytic processes. Compared with the existing reports, the present application has innovativeness and industrial application prospects.
2) Compared with the background technology, the present application has obvious progressiveness, aims at the drawbacks in the synthesis of 6-hydroxy-6-(hydroxymethyl)-2*H*-pyran-3(6*H*)-one, uses a heterogeneous catalyst, a cheap and environmental-friendly hydrogen peroxide as an oxidant and cleaner water as a reaction medium, and has mild reaction conditions, low cost, fewer byproducts, a yield of 95.2%, and environmental friendliness. The product is easy to separate and the catalyst can be reused. Therefore, the present application has high atom economy and green index, and has good industrial application prospects

### BRIEF DESCRIPTION OF THE DRAWINGS

In order to provide a clearer explanation of the embodiments or technical solutions in the present application, a brief introduction will be given to the accompanying drawings required in the embodiments or description of the prior art. It is evident that the accompanying drawings in the following description are only some of the embodiments recorded in the present application. For ordinary technical personnel in the art, other accompanying drawings can be obtained based on these drawings without any creative effort.
FIG. 1 is a product entity diagram of 6-hydroxy-6-(hydroxymethyl)-2*H*-pyran-3(6*H*)-one prepared in example 1 of the present application;
FIG.2 is a liquid chromatography-time-of-flight mass spectrometry coupled chromatogram of 6-hydroxy-6(hydroxymethyl)-2*H*-pyran-3(6*H*)-one prepared in example 10 of the present application;
FIG.3 is a liquid chromatography-time-of-flight mass spectrometry coupled mass spectrum of 6-hydroxy-6(hydroxymethyl)-2*H*-pyran-3(6*H*)-one prepared in example 10 of the present application; and
FIG. 4 is a UV absorption visible spectrum of 6-hydroxy-6(hydroxymethyl)-2*H*-pyran-3(6*H*)-one prepared in example 12 of the present application.

### DETAILED DESCRIPTION OF THE EMBODIMENTS

As mentioned above, in view of the drawbacks of the prior art in the synthesis of 6-hydroxy-6(hydroxymethyl)-2*H*-pyran-3(6*H*)-one, the inventor of this case, after long-term research and a lot of practice, proposed the technical scheme of the present application, which provides a method for synthesizing 6-hydroxy-6(hydroxymethyl)-2*H*-pyrano-3(6*H*)-one through efficient catalytic oxidization of 2,5-furan dimethanol under mild conditions by using the heterogeneous catalyst titanium silicon molecular sieve and cheap and environmentally friendly oxidant (hydrogen peroxide) and directly using clean water as the reaction medium, thereby achieving a cleaner and more economical catalytic process. Compared with the existing reports, the present application has innovativeness and industrial application prospects.

In the present application, the reaction principle for preparing bioactive molecules 6-hydroxy-6(hydroxymethyl)-2*H*-pyran-3(6*H*)-one through one-step catalytic oxidization of 2,5-furan dimethanol may lie in direct epoxidation of a double bond in a furan ring. The reaction formula is as follows: 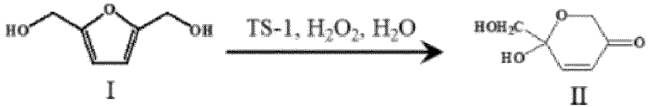 wherein, I is 2,5-furan dimethanol, and II is 6-hydroxy-6(hydroxymethyl)-2*H*-pyran-3(6*H*)-one; the reaction temperature is 15°C-100°C, and the reaction time is 20 min-120 min.

The following will provide further explanations and explanations on the technical solution, its implementation process, and principles.

An aspect of the embodiment of the present application provides a method for preparing 6-hydroxy-6(hydroxymethyl)-2*H*-pyran-3(6*H*)-one through catalytic oxidization under mild, green and environmental-friendly and efficient reaction conditions, comprising:

catalytically oxidizing 2,5-furan dimethanol for 20 min-120 min at 15°C-100°C by using titanium silicalite as a catalyst, hydrogen peroxide as an oxidant and water as a reaction medium so as to obtain 6-hydroxy-6(hydroxymethyl)-2*H*-pyran-3(6*H*)-one.

In some embodiments, the method for preparing 6-hydroxy-6(hydroxymethyl)-2*H*-pyran-3(6*H*)-one through catalytic oxidization comprises: obtaining 6-hydroxy-6(hydroxymethyl)-2*H*-pyran-3(6*H*)-one through a one-step catalytic oxidization process by using titanium silicalite as a catalyst, 2,5-furan dimethanol as a reaction raw material and water as reaction medium in the presence of oxidant hydrogen peroxide under mild conditions (react for 20 min-60 min at 15-70°C).

In some more specific embodiments, the method for preparing 6-hydroxy-6(hydroxymethyl)-2H-pyran-3(6*H*)-one through catalytic oxidization comprises:

evenly mixing 2,5-furan dimethanol, titanium silicalite, hydrogen peroxide and water, stirring the obtained reaction system to react for 20 min-60 min at 15°C-70°C, and performing one-step catalytic oxidization to obtain 6-hydroxy-6(hydroxymethyl)-2*H*-pyran-3(6*H*)-one.

In some preferred embodiments, a silica-to-titanium ratio in the titanium silicalite is (15-80): 1, preferably the silica-to-titanium ratio is (30-50):1.

In some preferred embodiments, a mass ratio of the catalyst titanium silicalite to the raw material 2,5-furan dimethanol is (0.1-20):1, preferably the mass ratio of the catalyst to the raw material is (0.4-5.0):1.

In some preferred embodiments, the reaction raw material 2,5-furan dimethanol (BHMF) is obtained by reducing 5-hydroxymethylfurfural.

In some preferred embodiments, the mass concentration of 2,5-furan dimethanol in the mixed reaction system is 1 g/L-50 g/L, preferably 5 g/L-25 g/L. That is to say, in the reaction medium water, the mass concentration of 2,5-furan dimethanol is 1 g/L-50 g/L, preferably, the concentration of the substrate is 5 g/L-25 g/L.

In some preferred embodiments, the oxidant hydrogen peroxide is a hydrogen peroxide aqueous solution with a mass concentration of 30%-50%.

In some preferred embodiments, the concentration of the oxidant in the mixed reaction system is 0.02 mol/L-10 mol/L, preferably 0.04 mol/L-2 mol/L. That is to say, in the reaction medium water, the concentration of the oxidant is 0.02 mol/L-10 mol/L, preferably the concentration is 0.04 mol/L-2 mol/L.

In some preferred embodiments, the temperature of the reaction is 15°C-100°C, preferably 25°C-70°C.

In some preferred embodiments, the time of the reaction is 20 min-120 min, preferably 30 min-60 min.

Further, the conversion rate of 2,5-furan dimethanol in the method is above 90%, and the yield of 6-hydroxy-6(hydroxymethyl)-2*H*-pyran-3(6*H*)-one is above 75%.

In conclusion, the method for synthesizing 6-hydroxy-6(hydroxymethyl)-2*H*-pyran-3(6*H*)-one through one-step efficient catalytic oxidization of 2,5-furan dimethanol under mild conditions by using heterogeneous catalyst titanium silicalite and inexpensive and an environmental-friendly oxidant (hydrogen peroxide) and directly using water as a reaction medium provided by the present application achieves a cleaner and more economical catalytic process, has low cost, fewer byproducts, a yield of 95.2%, easily product separation, environmentally friendliness, reused catalyst, has high atom economy and green index, and good industrial application prospects.

In order to make the purpose, technical solution and advantages of the present application clearer, the present invention will be further set forth in conjunction with specific embodiments and accompanying drawings. It should be understood that the specific embodiments described herein are only for explaining the present application, but experimental conditions and set parameters should not be considered as limiting the technical solution of the present application. Furthermore, the scope of protection of the present application is not limited to the following embodiments. In addition, technical features involved in various embodiments of the present application described hereinafter can be mutually combined without conflict.

Unless otherwise stated, raw materials and reagents used in embodiments of the present application are all commercially available.

### Example 1

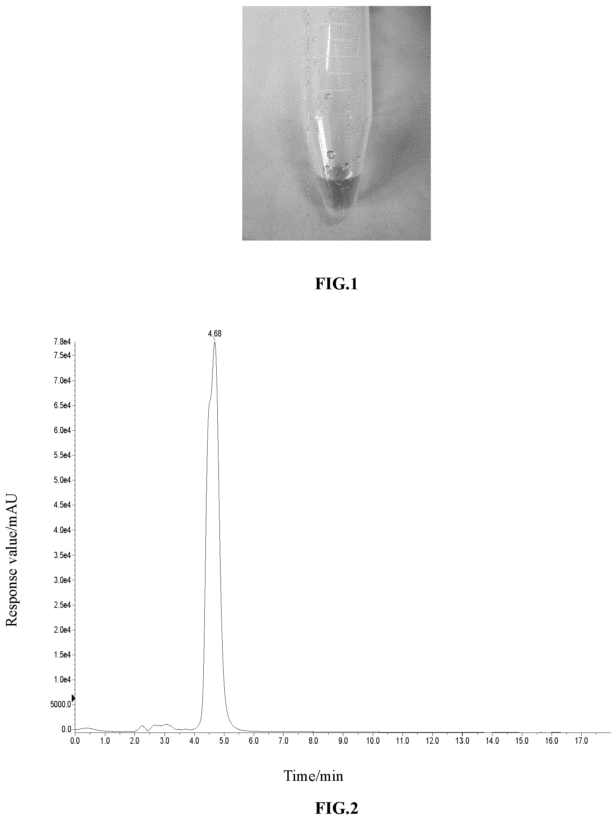

2,5-furan dimethanol (0.05g, 5 g/L), titanium silicalite TS-1 (a molar ratio of Si/Ti was 30:1, 0.1 g), H₂O₂ (30 wt%, 0.06 mol/L) were added into 10 mL of H₂O and stirred for 30 min at 30°C. After cooling, 5 mL of reaction solution was added into a 100 mL volumetric flask so that the total volume was 100 mL, and underwent high performance liquid chromatography determination after passing through a 0.22 µm microporous filter membrane. The conversion rate of 2,5-furan dimethanol was 100%, and the yield of 6-hydroxy-6(hydroxymethyl)-2*H*-pyran-3(6*H*)-one was 95.2%. By referring to FIG.1, the product is yellow oil.

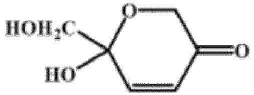

The NMR characterization data of the product 6-hydroxy-6(hydroxymethyl)-2*H*-pyran-3(6*H*)-one is as follows: 1H-NMR (400 MHz, CDCl₃): δ=6.84(d, 1H), 6.20(d, 1H), 4.63(d, 1H), 4.18(d, 1H), 3.82(d, 1H), 3.65(d, 1H).

### Examples 2-5

Other process conditions and reaction steps are the same as those in example 1, but different reaction temperatures are used. The results are seen in Table 1.

**Table 1 Experimental results for catalytic conversion of 2,5-furan dimethanol over titanium silicalite at different temperatures**

| Items | Reaction temperature (°C) | Conversion rate (%) of 2,5-furan dimethanol | Yield (%) of 6-hydroxy-6(hydroxymethyl)-2*H*-pyran-3(6*H*)-one |
|---|---|---|---|
| Example 2 | 15 | 96.8 | 87.6 |
| Example 3 | 25 | 100 | 94.5 |
| Example 4 | 70 | 100 | 91.3 |
| Example 5 | 100 | 100 | 85.2 |

### Examples 6-8

Other process conditions and reaction steps are the same as those in example 1, but different reaction times are used. The results are seen in Table 2.

**Table 2 Experimental results of catalytic conversion of 2,5-furan dimethanol over titanium silicalite at different temperatures**

| Items | Reaction time (min) | Conversion rate (%) of 2,5-furan dimethanol | Yield (%) of 6-hydroxy-6(hydroxymethyl)-2*H*-pyran-3(6*H*)-one |
|---|---|---|---|
| Example 6 | 20 | 96.7 | 79.8 |
| Example 7 | 60 | 100 | 93.1 |
| Example 8 | 120 | 100 | 90.4 |

### Examples 9-11

Other process conditions and reaction steps are the same as those in example 1, but different catalyst amounts are used, that is, mass ratios of titanium silicalite to 2,5-furan dimethanol are different. The results are seen in Table 3.

**Table 3 Experimental results of catalytic conversion of 2,5-furan dimethanol over titanium silicalite under different catalyst amounts**

| Items | A mass ratio of titanium silicalite to 2,5-furan dimethanol | Conversion rate (%) of 2,5-furan dimethanol | Yield (%) of 6-hydroxy-6(hydroxymethyl)-2*H*-pyran-3(6*H*)-one |
|---|---|---|---|
| Example 9 | 0.1 | 90.3 | 76.8 |
| Example 10 | 0.4 | 100 | 93.6 |
| Example 11 | 20 | 100 | 88.2 |

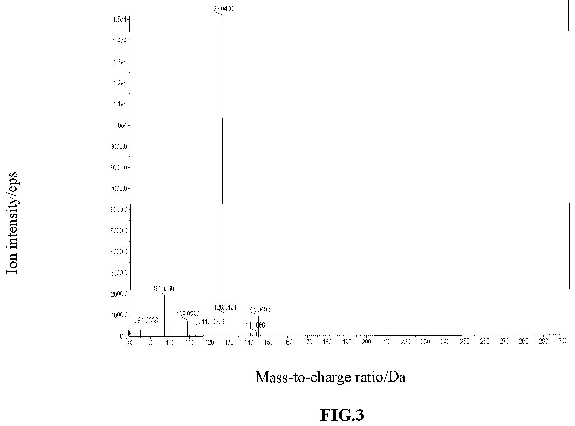

The liquid chromatography-time-of-flight mass spectrometry coupled chromatogram of 6-hydroxy-6(hydroxymethyl)-2*H*-pyran-3(6*H*)-one prepared in example 10 refers to FIG.2, and a mass spectrum of 6-hydroxy-6(hydroxymethyl)-2*H*-pyran-3(6*H*)-one refers to FIG.3.

### Examples 12-14

Other process conditions and reaction steps are the same as those in example 1, but different substrate mass concentrations are used. The results are seen in Table 4.

**Table 4 Experimental results of catalytic conversion of 2,5-furan dimethanol over titanium silicalite at different substrate mass concentrations**

| Items | 2,5-furan dimethanol | Conversion rate (%) of 2,5-furan dimethanol | Yield (%) of 6-hydroxy-6(hydroxymethyl)-2*H*-pyran-3(6*H*)-one |
|---|---|---|---|
| Example 12 | 1 | 100 | 89.7 |
| Example 13 | 20 | 100 | 92.8 |
| Example 14 | 50 | 92 | 78.4 |

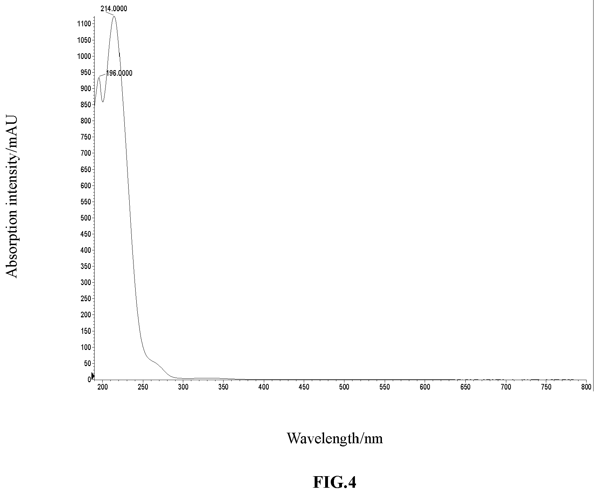

The UV absorption visible spectrogram of 6-hydroxy-6(hydroxymethyl)-2*H*-pyran-3(6*H*)-one prepared in example 12 refers to FIG.4.

### Examples 15-17

Other process conditions and reaction steps are the same as those in example 1, but different oxidant hydrogen peroxide concentrations are used. The results are seen in Table 5.

**Table 5 Experimental results of catalytic conversion of 2,5-furan dimethanol over titanium silicalite at different hydrogen peroxide concentrations**

| Items | Concentration of hydrogen peroxide | Conversion rate (%) of 2,5-furan dimethanol | Yield (%) of 6-hydroxy-6(hydroxymethyl)-2*H*-pyran-3(6*H*)-one |
|---|---|---|---|
| Example 15 | 0.02 | 90.5 | 77.4 |
| Example 16 | 2 | 100 | 91.1 |
| Example 17 | 10 | 100 | 83.4 |

### Examples 18-20

Other process conditions and reaction steps are the same as those in example 1, but titanium silicalite catalysts with different silica-to-titanium ratios are used. The results are seen in Table 6.

**Table 6 Experimental results of catalytic conversion of 2,5-furan dimethanol over titanium silicalite in different silica-to-titanium ratios**

| Items | Titanium silicalite with different silica-to-titanium ratios | Conversion rate (%) of 2,5-furan dimethanol | Yield (%) of 6-hydroxy-6(hydroxymethyl)-2*H*-pyran-3(6*H*)-one |
|---|---|---|---|
| Example 18 | 15 | 92.7 | 75.3 |
| Example 19 | 50 | 100 | 92.4 |
| Example 20 | 80 | 96.2 | 80.7 |

### Comparative example 1

By comparing control example disclosed in Synth. Commun., 2015, 45, 348-354 with example 1, the difference was that the oxidant used was m-chloroperoxybenzoic acid. In this control example, when 2,5-furan dimethanol was completely converted, the yield of 6-hydroxy-6(hydroxymethyl)-2*H*-pyran-3 (6*H*)-one is not ideal, only 45%-63%.

### Comparative example 2

CN 109180624 A disclosed a preparation method of a 2-substituted-6-hydroxy-2H-pyran-3-one compound, in which an organic solvent was used. Furthermore, cumene hydroperoxide was used as an oxidant, this preparation was carried out in the presence of a triisopropyl oxyvanadate homogeneous catalyst, but the yield was only 61%-78%.

This control example did not involve conversion of 2,5-furan dimethanol, and the target product of the present application was not produced.

By virtue of the above technical solution, in the present application, 6-hydroxy-6(hydroxymethyl)-2*H*-pyran-3(6*H*)-one was obtained through a one-step catalytic oxidization process under mild conditions, so as to achieve a cleaner and economic catalysis process. The present application has a yield as high as 95.2%, is low in cost, less in byproduct and environmental-friendly, and has easily separated product and good industrial application prospects.

Various aspects, embodiments, features and examples of the present application should be deemed as being illustrative in all aspects and are not intended to limit the present application, and the scope of the present application is only defined by claims. Those skilled in the art will understand other embodiments, modifications and uses without departing from the spirit and scope claimed by the present application.

Throughout the present application, when a composition is described as having, comprising, or including specific components, or when a process is described as having, comprising, or including specific process steps, it is expected that the composition taught in the present application will also be substantially composed of or composed of the described components, and the process taught in the present application will also be mainly composed of or composed of the described process steps.

Unless otherwise specifically stated, the use of the terms "include, includes, including" and "have, has or having" should generally be understood as being opened and not limiting.

It should be understood that the order of each step or the order in which specific actions are performed is not very important, as long as the instructions in this application remain operable. In addition, two or more than two steps or actions can be simultaneously performed.

In addition, the inventor of this case also conducted experiments using other raw materials, process operations, and process conditions described in this manual, referring to the aforementioned embodiments 1-20. They also obtained 6-hydroxy-6(hydroxymethyl) -2*H*-pyran-3(6*H*)-one with high selectivity.

Although the present application has been described with reference to illustrative embodiments, those skilled in the art will understand that various other changes, omissions, and/or additions may be made without departing from the spirit and scope of the present application, and substantial equivalents may be used to replace the components of the embodiments. In addition, many modifications can be made without departing from the scope of the present application to adapt specific situations or materials to the teachings of the present application. Furthermore, unless specifically stated, any use of terms first, second, etc. does not indicate any order or importance, but rather uses terms first, second, etc. to distinguish one element from another.

## Claims

1. A method for preparing 6-hydroxy-6(hydroxymethyl)-2*H*-pyran-3(6*H*)-one through catalytic oxidization, comprising:
catalytically oxidizing 2,5-furan dimethanol for 20 min-120 min at 15°C-100°C by using titanium silicalite as a catalyst, hydrogen peroxide as an oxidant and water as a reaction medium, so as to obtain 6-hydroxy-6(hydroxymethyl)-2*H*-pyran-3(6*H*)-one.

2. The method according to claim 1, comprising: evenly mixing 2,5-furan dimethanol, titanium silicalite, hydrogen peroxide and water, stirring the obtained reaction system to react for 20 min-60 min at 15°C-70°C, and performing one-step catalytic oxidization to obtain 6-hydroxy-6(hydroxymethyl)-2*H*-pyran-3(6*H*)-one.

3. The method according to claim 1 or 2, wherein a silica-to-titanium ratio in the titanium silicalite is (15-80): 1, preferably (30-50): 1.

4. The method according to claim 1 or 2, wherein a mass ratio of the titanium silicalite to 2,5-furan dimethanol is (0.1-20): 1, preferably (0.4-5.0): 1.

5. The method according to claim 1 or 2, wherein the 2,5-furan dimethanol is obtained by reducing 5-hydroxymethylfurfural.

6. The method according to claim 2, wherein the mass concentration of 2,5-furan dimethanol in the mixed reaction system is 1 g/L-50 g/L, preferably 5 g/L-25 g/L.

7. The method according to claim 1 or 2, wherein the oxidant is a hydrogen peroxide aqueous solution with a mass concentration of 30%-50%.

8. The method according to claim 1 or 2, wherein the concentration of the oxidant in the mixed reaction system is 0.02 mol/L-10 mol/L, preferably 0.04 mol/L-2 mol/L.

9. The method according to claim 1 or 2, wherein the temperature of the reaction is 25°C-70°C; and/or the time of the reaction is 30 min-60 min.

## Patentansprüche

1. Verfahren zur Herstellung von 6-Hydroxy-6(Hydroxymethyl)-2*H*-Pyran-3(6*H*)-one durch katalytische Oxidation, umfassend:
katalytisches Oxidieren von 2,5-Furan-Dimethanol für 20 Min. bis 120 Min. bei 15 °C bis 100 °C unter Verwendung von Titansilikalit als Katalysator, Wasserstoffperoxid als Oxidationsmittel und Wasser als Reaktionsmedium, um 6-Hydroxy-6(Hydroxymethyl)-2*H-*Pyran-3(6*H*)-one zu erhalten.

2. Verfahren nach Anspruch 1, umfassend: gleichmäßiges Mischen von 2,5-Furan-Dimethanol, Titansilikalit, Wasserstoffperoxid und Wasser, Verrühren des erhaltenen Reaktionssystems, um 20 Min. bis 60 Min. lang bei 15 °C bis 70 °C zu reagieren, und Durchführen einer einstufigen katalytischen Oxidation, um 6-Hydroxy-6(Hydroxymethyl)-2*H-*Pyran-3(6*H*)-one zu erhalten.

3. Verfahren nach Anspruch 1 oder 2, wobei das Siliciumoxid-Titan-Verhältnis im Titansilikalit (15-80): 1 beträgt, vorzugsweise (30-50): 1.

4. Verfahren nach Anspruch 1 oder 2, wobei ein Massenverhältnis des Titansilikalits zu 2,5-Furan-Dimethanol (0,1-20):1 beträgt, vorzugsweise (0,4-5,0):1.

5. Verfahren nach Anspruch 1 oder 2, wobei das 2,5-Furan-Dimethanol durch Reduktion von 5-Hydroxymethylfurfural erhalten wird.

6. Verfahren nach Anspruch 2, wobei die Massenkonzentration von 2,5-Furan-Dimethanol im gemischten Reaktionssystem 1 g/l bis 50 g/l beträgt, vorzugsweise 5 g/l bis 25 g/l.

7. Verfahren nach Anspruch 1 oder 2, wobei das Oxidationsmittel eine wässrige Wasserstoffperoxidlösung mit einer Massenkonzentration von 30 % bis 50 % ist.

8. Verfahren nach Anspruch 1 oder 2, wobei die Konzentration des Oxidationsmittels im gemischten Reaktionssystem 0,02 mol/l bis 10 mol/l beträgt, vorzugsweise 0,04 mol/l bis 2 mol/l.

9. Verfahren nach Anspruch 1 oder 2, wobei die Temperatur der Reaktion 25 °C bis 70 °C beträgt und/oder die Reaktionszeit 30 Min. bis 60 Min. beträgt.

## Revendications

1. Procédé de préparation de 6-hydroxy-6(hydroxyméthyl)-2*H*-pyran-3(6*H*)-one par oxydation catalytique, comprenant :
l'oxydation catalytique du 2,5-furane diméthanol pendant 20 min à 120 min à 15°C à 100°C en utilisant de la silicalite de titane comme catalyseur, du peroxyde d'hydrogène comme oxydant et de l'eau comme milieu réactionnel, de manière à obtenir du 6-hydroxy-6(hydroxyméthyl)-2*H*-pyran-3(6*H*)-one.

2. Procédé selon la revendication 1, comprenant : le mélange uniforme de 2,5-furane diméthanol, de silicalite de titane, de peroxyde d'hydrogène et d'eau, l'agitation du système réactionnel obtenu pour réagir pendant 20 min à 60 min à 15°C à 70°C et la réalisation d'une oxydation catalytique en une seule étape pour obtenir du 6-hydroxy-6(hydroxyméthyl)-2*H-*pyran-3(6*H*)-one.

3. Procédé selon la revendication 1 ou 2, dans lequel un rapport de la silice au titane dans la silicalite de titane est (15-80) : 1, de préférence (30-50) : 1.

4. Procédé selon la revendication 1 ou 2, dans lequel un rapport massique de la silicalite de titane au 2,5-furane diméthanol est (0,1-20) : 1, de préférence (0,4-5,0) : 1.

5. Procédé selon la revendication 1 ou 2, dans lequel le 2,5-furane diméthanol est obtenu par réduction du 5-hydroxyméthylfurfural.

6. Procédé selon la revendication 2, dans lequel la concentration massique du 2,5-furane diméthanol dans le système réactionnel mélangé est de 1 g/L à 50 g/L, de préférence de 5 g/L à 25 g/L.

7. Procédé selon la revendication 1 ou 2, dans lequel l'oxydant est une solution aqueuse de peroxyde d'hydrogène ayant une concentration massique de 30% à 50%.

8. Procédé selon la revendication 1 ou 2, dans lequel la concentration de l'oxydant dans le système réactionnel mélangé est de 0,02 mol/L à 10 mol/L, de préférence 0,04 mol/L à 2 mol/L.

9. Procédé selon la revendication 1 ou 2, dans lequel la température de la réaction est de 25°C à 70°C ; et/ou le temps de la réaction est de 30 min à 60 min.
